(19) Europäisches Patentamt
European Patent Office
Office européen des brevets

(11) EP 3 287 191 A1

(12) **EUROPEAN PATENT APPLICATION**

(43) Date of publication:
**28.02.2018 Bulletin 2018/09**

(21) Application number: **16185958.2**

(22) Date of filing: **26.08.2016**

(51) Int Cl.:
***B01J 23/26*** *(2006.01)*
***B01J 23/62*** *(2006.01)*
***B01J 35/00*** *(2006.01)*
***B01J 37/02*** *(2006.01)*
***B01J 38/12*** *(2006.01)*
***B01J 38/10*** *(2006.01)*
***B01J 23/96*** *(2006.01)*
***C07C 5/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Friedrich-Alexander-Universität Erlangen-Nürnberg**
**91054 Erlangen (DE)**

(72) Inventors:
- **Debuschewitz, Jonas**
  **91052 Erlangen (DE)**
- **Görling, Andreas**
  **82061 Neuried (DE)**
- **Steinrück, Hans-Peter**
  **91056 Erlangen (DE)**
- **Taccardi, Nicola**
  **90766 Fürth (DE)**
- **Wasserscheid, Peter**
  **91054 Erlangen (DE)**

(74) Representative: **Grimm, Siegfried**
**E. Blum & Co. AG**
**Vorderberg 11**
**8044 Zürich (CH)**

# (54) CATALYTICALLY ACTIVE COMPOSITIONS OF MATTER

(57) The present invention pertains to the field of catalyst and catalytic reactions. Specifically, the invention provides for new catalytically active compositions of matter, to methods of manufacturing them and to the use of such compositions.

EP 3 287 191 A1

## Description

The present invention pertains to the field of catalyst and catalytic reactions. Specifically, the invention provides for new catalytically active compositions of matter, to methods of manufacturing them and to the use of such compositions. The catalytically active compositions comprise an alloy which is liquid under reaction conditions and which is adsorbed on a support material.

Catalytically active compositions for commercial applications, comprising a support material and a catalytic active component, are well known.

J. Sattler et al. (Chemical Reviews, 2014, 114, 10613-10653) reviews the catalytic dehydrogenation of light alkanes on metals and metal oxides. The authors discuss commercial catalysts, suitable reaction conditions, mechanisms and drawbacks of current catalysts. Industrial applied catalyst systems for the dehydrogenation of short-chain alkanes, as for example n-butane, are platinum particles on alumina supports (Olefex process) and $CrzO_3$ on alumina supports (Catofin process). As main drawbacks, selectivity, conversion rate and deactivation (mainly caused due to coke formation) are identified.

S. Veldurthi et al. (Catalysis Today, 2012, 185, 88-93) discuss promotional effects of Cu on Pt / alumina and Pd / alumina catalysts during dehydrogenation. The authors show that such modification may beneficially influence selectivity and in some instances activity of the catalyst. S. Bocanegra et al. (Catalysis Today, 2009, 143, 334-340) discuss highly selective and stable bimetallic catalysts supported on different materials for n-butane dehydrogenation. The authors show that the support influences catalyst performance.

J. Sattler et al. (Physical Chemistry Chemical Physics, 2013, 12095-12103] study the deactivation of Pt/A1203 and Pt-Sn/A1203 propane dehydrogenation catalysts. The authors conclude that addition of hydrogen beneficially influences catalyst performance and decreases the formation of coke deposits.

In consequence, there is a need for improved catalysts, resolving one or more of the shortcomings discussed in the prior art.

Thus, it is an object of the present invention to mitigate at least some of these drawbacks of the state of the art. In particular, it is an aim of the present invention to provide catalytic systems with improved activity and / or selectivity and / or less deactivation.

These objectives are achieved by the catalytically active compositions as defined in claim 1 and by the catalytic processes as defined in claim 12. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending on the specific embodiment, selected definitions, embodiments or ranges may not apply.

Unless otherwise stated, the following **definitions** shall apply in this specification:

As used herein, the term "**a**", "an", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, non-limiting sense.

The present invention will be better understood by reference to the **figures.**

**Figs. 1-3** show the results of test runs using diluted butane as starting material. Conversion, TOF, and selectivity are shown vs. time. The results obtained using the inventive catalytic material are indicated with solid symbols.

**Figs. 4-5** show the results of test runs using pure (non-diluted) butane as starting material. TOF and selectivity are shown vs. time. The results obtained using the inventive catalytic material are indicated with solid symbols.

**Fig. 6** compares the catalytic activity (TOF*) of n-butane dehydrogenation with non-diluted starting material, where solid circle and solid triangle represent commercial CrAlOx- and Pt/AlOx-dehydriation catalysts. The inventive catalytic material is represented by a solid square.

**Fig 7:** shows catalytic activity (TOF) of n-butane dehydrogenation with non-diluted starting material in a long-term experiment over 110hrs.

**Fig 8-9** show the results of test runs using catalysts with further transition metals and pure (non-diluted) butane as starting material, where solid circles represent a rhodium containing catalyst, solid triangles represent a platinum

containing catalyst and solid squares represent a chromium containing catalyst. TOF and selectivity are shown vs. time.

**Figure 10:** shows a flow scheme of the continuous flow dehydrogenation rig used for the catalytic reaction testing. "MFC" represents the mass flow controller for the starting materials, namely inert gas (identified as "Helium") and feedstock (identified as "n-Butane"). "TIC" represents temperature indicator controllers placed in the reactor and "GC" represents analytical devices for product characterization (specifically Gas Chromatograph).

In more general terms, in a **first aspect**, the invention relates to a new composition of matter comprising a support and a coating, whereby said support contains a non-metallic and porous component (A), and said coating comprises a first metallic component (B) and a second metallic component (C), characterized in that said component (C) is catalytically active and said component (C) is present in component (B) in dissolved and / or in suspended form, and characterized in that said coating (B+C) has a melting point below 200°C.

The compositions disclosed herein are catalytically active and may therefore also be considered as catalytic systems or catalyst compositions.

Key features of the present invention are that component (C) is present in component (B) in dissolved and / or in suspended form (thereby forming an alloy of (B+C)), and that (B+C) has a melting point below 200°C. In use, a liquid alloy of components (B) and (C) is formed in the inventive catalyst composition. This liquid alloy is present on said support material. These features distinguish the inventive catalyst compositions from known catalyst compositions, where the catalytic active material (C) is present in solid form. Without being bound to theory, it is believed that these features result in the improvements observed, namely higher reactivity, higher selectivity, less deactivation. In its broadest sense, the invention thus provides for the use of an alloy comprising components (B+C) which is located on a support comprising component (A) in catalytic reactions, whereby said alloy is liquid under the conditions of said catalytic reaction. The inventive composition of matter thus benefits from the cooperative effect of components (A), (B) and (C). While (A) provides for the well-known function of a support and (C) provides for the well-known function of a catalyst site, component (B) provides for a liquid environment of the catalyst (C). It was unexpected, that the component (C) retains its catalytic function in such liquid environment, despite the teachings of the prior art which suggest the importance of crystalline structures (see e.g. Sattler et al, cited above, figs. 12 and 20). Moreover, and explained in further detail below, the catalyst become more active, more selective and show less deactivation when compared to the known catalysts.

This aspect of the invention shall be explained in further detail below:

**Support:** Support materials, also termed carriers, are generally known and may be selected by the skilled person according to the catalysed reaction. The support contains, i.e. comprises or consists of, component (A). Accordingly, such support materials may comprise promotors or other components to increase stability, or otherwise beneficially influence catalysis. Support materials are generally not catalytically active themselves.

The support can be formed in any suitable shape, such as a sphere, cylinder, tablet, powder and the like. In a preferred embodiment the catalyst carrier is a sphere or a cylinder. The spherical or cylindrical catalyst carrier can be formed in any suitable size, for a spherical catalyst carrier preferably from about 1 to about 30 millimetres in diameter. Alternatively, the support can be formed as a monolithic structures adapted to a specific housing, preferably with sizes of 10 cm or more in one dimension. Accordingly, the invention provides in advantageous embodiments for a composition as described herein, wherein said support is

- in the form of a powder, particularly with particle size in the range of 10 to 800 $\mu$m;
- in the form of granulated material ("granules"), such as pellets or beads, particularly with a particle size of 50 to 5000 $\mu$m;
- in the form of spheres, such as beads or cylinders, particularly with particle size of 0.5-50 mm such as 1 to 30 mm;
- in the form of a shaped article, such as a porous monolith, preferably with sizes of 10 cm or more in one dimension.

**Coating:** Components (B) and (C) are located on the surface of the support, thereby coating component (A). Without being bound to theory, it is believed that the liquid alloy comprising components (B) and (C) is adsorbed on support (A).

In one embodiment, components (B+C) fully cover said support, thereby forming a continuous film. The film thickness may vary, but typically is in the range of at least 0.4 nm. Maximum film thickness is determined by the loading of (B+C), support surface and total pore volume of the support and is considered less critical.

In one alternative embodiment, components (B+C) partly cover said support, thereby forming a discontinuous coating or droplets. It was found that catalyst compositions are suitable where at least 1 %, preferably at least 10 % of the support are covered with component (B+C).

**Component (A):** Suitable support materials (A) may be selected from the group consisting of metal oxides, silicates, zeolites, carbonaceous materials. Such materials are known in the field and are commercially available or may be obtained according to known processes. Metal oxides include alumina, silica-alumina, zinc oxide, nickel spinel, titania, zirconia, ceria, chromia-alumina, magnesium oxide, cerium oxide and mixtures thereof. The preferred metal oxide support is alumina.

Silicates include all salts and esters of Si(OH)4 and their condensates. These compounds are composed of SiO4 tetrahedrons that are connected among each other in various ways. Unconnected position in the structure of silicates carry alkali ions for charge compensation or form hydroxide terminations. The term silicates thus includes the parent compound SiO2. The preferred silicate support is a porous glass with a majority of its pores being above 2 nm pore size.

Zeolites include naturally occurring zeolites and synthetic zeolites and combinations thereof. Zeolites may or may not comprise acidic sites. In exemplary embodiments, the zeolite is selected from the group consisting of ferrierite, zeolite beta, Y zeolite, mordenite, MCM-22, ZSM-23, ZSM-57, SUZ-4, EU-1, ZSM-11, (S)A1P0-31, SSZ-23, SAPO, ALPA, MeALPO and ZSM-5.

Carbonaceous materials include porous carbon materials obtained synthetically from pyrolysis of carbon containing precursors such as tar, coconut or organic polymers. These porous carbon materials may be activated by treatments with water vapour or carbon dioxide at elevated temperatures. The preferred carbonaceous support contains a pore size distribution that offers more than 5% of its pore volume in form of pores with a pore diameter larger than 2 nm.

Component (A) preferably is a high surface material (such as a porous material), to provide increased surface area for the catalytic reactions. Thus, the specific surface may vary over a broad range. Suitable support materials may have a specific surface area (SSA) of 2-10000 $m^2/g$, preferably 50-1000$m^2/g$. SSA may be determined according to BET methods.

Metal oxides, silicates and carbonaceous materials, as discussed herein, are typically considered as high surface area support materials for many catalytic reactions, in case the support has a surface area greater than about 150 m2/g, preferably from about 150 to about 1000 m2/g, more preferably from about 175 to about 500 m2/g, and most preferably from about 200 to about 300 m2/g. The pore volume of the metal oxides, silicates and carbonaceous materials, as discussed herein, may vary over a broad range, but is preferably in the range of about 0.2 to about 0.6 cc/g.

Zeolites are typically considered porous, suitable for many catalytic reactions in case the pores are selected such that they are large enough to allow access by large molecules in the feedstock.

Component (A) preferably is thermally stable. Suitable support materials may be selected by the skilled person according to the catalysed reaction and are typically thermally stable up to at least 400°C, preferably up to at least 800°C.

**Component (B):** As component (B) low melting metals and alloys are suitable. Advantageously, component (B) has a melting point below 200°C.

It is apparent that component (B) is selected not to inhibit catalytic activity of component (C). Suitable metals are selected from the group consisting of Ga (melting point= 30 °C), In (melting point= 157 °C), Hg (melting point= -39 °C) and mixtures thereof. Suitable alloys are selected from gallium-indium eutectics (melting point down to 15 °C), Wood's metal, a mixture of bismuth-lead-cadmium-tin with melting points down to 60 °C, and Rose's metal, a mixture of bismuth-lead-tin with melting points down to 94 °C.

**Component (C):** As component (C), any metal suitable for a given catalytic reaction may be used. Preferred are metals (C) that dissolve in component (B) to at least 1 wt%, preferably at least 10wt% at temperatures above 200°C.

In the context of this invention, component (C) is catalytically active, if the reaction to be catalysed occurs at that component (C), or if component (C) induces catalytic properties in component (B). In other words, the catalytic mechanism is not relevant. Any component (C), which is either catalytically active or which promotes catalytic activity, may be used according to this invention.

Suitable components (C) selected from the group of metals as defined by IUPAC group 3 to 12, particularly platinum group metals; and from the group of alloys comprising a metal as defined by IUPAC group 3 to 12, particularly alloys comprising platinum group metals.

In one embodiment, component (C) is selected from the group consisting of Ti, Zr, Cr, Mo, W, Rh, Fe, Ru, Co, Rh, Ir, Ni, Pd Pt, Cu, Ag, Au and its mixtures.

In a preferred embodiment, component (C) is selected from the group consisting of Pt, Pd, Ru, Rh, Ir, Ni, Fe, Cr, Cu, Co and its mixtures.

In a further preferred embodiment, component (C) is selected from the group alloys consisting of Pt/Co, Pt/Sn, Pt/Re, Pd/Zn.

In a particular preferred embodiment, component (C) is selected from Pd, Pt, Rh and Cr.

The amount of (B) and (C) present on the catalyst composition may vary over a broad range; suitable ranges may be determined in routine experiments, dependent on the catalytic reaction and the reactor design. Typical ranges for the amount [(C)+(B)]:(A) are from about 0.01 to about 300 weight percent, preferably 0.05 to 50 weight percent, based on the total weight of the support.

The amount of (B) to (C) may also vary over a broad range; suitable ranges may be determined in routine experiments, dependent on the catalytic reaction and the reactor design. Typical ranges for the amount (B):(C) are from about 1 :1 to 1000:1, preferably 5:1 to 100: 1, such as 10:1 to 20:1.

In a **second aspect**, the invention relates to a process for manufacturing a composition as described herein. The components (B) and (C) can be introduced into the support (A) by any conventional procedure which produces the proper metal loading. Such methods are known per se but not yet applied to the specific combination of components (A), (B) and (C).

This aspect of the invention shall be explained in further detail below:

One preferred technique involves impregnating the support with a solution comprising a precursor of component (B) followed by solvent removal to obtain a coated material.

This coated material is than subjected to a second impregnation step where a solution comprising a precursor of component (C) is contacted with the initially obtained coated material. After solvent removal, and optionally further activation steps, the inventive composition is obtained. Accordingly, the invention provides for a method for manufacturing a composition as described herein, comprising the steps of

(i) providing a component (A), a liquid composition comprising a precursor of component (B) and a liquid composition comprising a precursor of component(C);
(ii) contacting component (A) with said liquid composition of (B) and removing solvent;
(iii) contacting the thus obtained materials with said liquid composition of (C) and removing solvent;
(iv) optionally further activation steps;

to thereby obtain said solid composition of matter.

An alternative technique involves impregnation with a liquid composition comprising both, first precursor of (B) and second precursor of (C) in one single impregnation step. Accordingly, the invention provides for a method for manufacturing a composition as described herein, comprising the steps of

(i) providing a component (A) a liquid composition comprising a precursor of component (B) and of component (C);
(ii) contacting component (A) with said liquid compositions comprising (B) and (C) and removing solvent;
(iii) optionally further activation steps;

to thereby obtain said solid composition of matter.

Suitable precursors of component (B) and of component (C) are known and available by known methods. As an exemplary embodiment, the class of alkylammnonium gallates is identified as precursor of component (B).

As an exemplary embodiment, the classes of alkylammonium halometallates and of ammonium halometalattes are identified as precursor of component (B).

As outlined above, the compositions obtained according to the methods described herein have outstanding catalytic properties. The invention therefore also relates to a solid composition, particularly a catalyst composition, obtained by a method as described herein.

In a **third aspect**, the invention relates to the use of compositions as described herein, particularly to catalytic reactions employing the compositions described herein. It has been surprisingly discovered that high performing catalysts, particularly selective dehydrogenation catalyst, are obtained when a combination of components (B) and (C) is coated on a high surface area inert support (A). Unexpectedly, the inventive catalyst systems are less prone to coking, thereby showing less deactivation. This allows for reduced regeneration times and longer reaction times when compared to known catalyst compositions.

This aspect of the invention shall be explained in further detail below:

In use, the inventive catalyst composition is placed in a reactor. The inlet temperature of the feed stream in the reactor is raised to a level sufficient to perform the reaction and is above the melting temperature of component (B). Generally, this temperature is above 200°C. Further, the temperature is chosen to ensure integrity of the support material. This temperature depends on the support, but typically is below 1500°C, such as below 1000°C.

In one embodiment, the invention provides for the use of a composition as described herein (first aspect), as a catalyst, particularly as a catalyst in a gas-phase reaction. In such gas-phase reactions, any suitable reaction pressure can be used. Generally, the total pressure in the reactor is in the range between 1 bar and 100 bar, preferably between 1 bar and 30 bar. In such gas-phase reactions, the gas hourly space velocity (GHSV) may vary over a broad range, but typically is of about 100 to about 100.000 liters per liter of catalyst per hour.

In one embodiment, the invention provides for the use of a composition as described herein (first aspect), where said catalysed reaction is selected from the group of endothermic reactions. Endothermic reactions are typically performed at higher temperatures, to beneficially influence the reaction equilibrium. Such high temperatures in turn favour side reactions, such as coke deposition on the catalyst. The inventive catalyst system allows higher reaction temperatures while still avoiding coke formation and thereby deactivation of catalyst. As a result, reaction cycles may be more than 1 h, preferably more than 100 h before regeneration becomes necessary.

Accordingly, the invention provides for a catalytic process, comprising the step of contacting an organic starting material with a composition described herein (first aspect), where

- reaction temperatures are above 200°C; and/or
- reaction cycles are at least 1 hour; and/or
- starting materials and reaction products are gaseous under reaction conditions.

A broad range of catalytic reactions may benefit from the inventive catalyst compositions. Due to the high reaction temperatures, endothermic reactions are preferred. Endothermic reactions shall include both (i) reactions where all reaction steps are endothermic and (ii) reactions where some reaction steps are exothermic but the over all reaction being endothermic. Moreover, slightly exothermic reactions may still benefit from the inventive catalytic systems and are thus also included. Preferably the catalytic process described herein is selected from the group of

- catalytic isomerisation of hydrocarbons or of aromatics,
- catalytic dehydrogenation of hydrocarbons,
- catalytic reforming.

These catalytic processes are known per se but not yet performed with the catalyst compositions described herein.

Preferred feedstocks for the above described catalytic processes include aliphatic and aromatic hydrocarbons, including C2+ alkanes (such as linear, branched and cyclic C2-24 alkanes) and C6+ aromatics, such as C6-14 arenes and C6-C14 arenes subsstitued with one or more C1-4 alkyl groups). Specific feedstocks include ethane, propane, butane, isobutane, pentane, isopentanes, hexane, isohexanes, cyclohexane, heptane, isoheptanes, methylcyclohexane, ethyl benzene, perhydro benzyltoluene, perhydro dibenzyltoluene and mixtures thereof.

A particularly preferred reaction is the dehydrogenation of hydrocarbons, particularly of the hydrocarbons cited above. As discussed above, existing catalysts do not perform consistently well, particularly in terms of conversion rate, selectivity and stability. By the process of this invention, these issues are successfully addressed as outlined in the examples and supported by the figures 1-9. Specifically, long-term stability over more than 100 h is observed (fig. 7) while the prior art reports stability of commercial catalysts being excellent when regeneration becomes necessary after 7 h (Sattler, cited above, chapter 2.3). Further, catalytic activity (indicated as TOF) and selectivity of the inventive catalyst composition is consistently higher compared to the commercial catalysts (figs. 2-5).

Regeneration of the inventive catalyst composition, once required, may be accomplished according to standard procedures, e.g. by heating the catalyst composition in air or hydrogen, to thereby burn off any deposited material, such as coke.

The amount of catalyst used for a specific catalytic reaction may vary over a broad range. Typically, the amount of the inventive catalyst for a given catalytic reaction is in the same range or lower as the amount of catalyst currently used in such reactions.

Without being bound to theory, it is believed that at least three cooperating effects provide for the excellent catalytic properties disclosed herein.

First, the liquid nature of alloy (B+C), as present under reaction conditions, provides for a highly dynamic behaviour on the fluid-fluid interface and a continuous regeneration of the alloy surface under reaction conditions. This highly dynamic behaviour is likely to supresses coking.

Second, component (C), although present in small amounts, provides for a high catalytic activity. This may be attributed to an enrichment of (C) on the liquid alloy surface and thereby facilitates contact to the feedstock. As a third effect, the high surface area provided by the support and the low vapour pressure of the alloy (B+C) provides for a large contact area of feedstock to catalytic active material.

To further illustrate the invention, the following **examples** are provided. These examples are provided with no intend to limit the scope of the invention.

## 1. Catalyst Preparation

### 1.1 Pd/Ga decorated material

**Preparation of $(Et_3N)GaH_3$**

This compound was synthetized using the procedure described by Shriver et Al. (Inorg. Synth. 1977, 17, 42-45) using triethylammonium chloride (Sigma-Aldrich) instead of trimethylammonium. The compound was not isolated and used as ethereal solution.

**Preparation of $(NH_4)2[PdCl_4]$**

A 10 mM solution of $(NH_4)_2[PdCl_4]$ was prepared as follows: $PdCl_2$ (171.5 mg, 1 mmol, Sigma-Aldrich) and $NH_4Cl$ (108.3 mg, 2 mmol, Sigma-Aldrich) were added to ca. 30 mL of distilled water in a beaker. The resulting suspension was boiled until it became homogeneous, then cooled and transferred to a 100 mL calibrated flask and diluted with distilled water.

**Preparation of Ga decorated porous glass**

10 g of porous glass (non-treated SiO2, available as trisopor, Biosearch Technologies) were heated under vacuum (1 mbar) at 300 °C overnight in a Schlenk flask. After cooling the system, they were suspended in 20 mL dry diethyl ether, under argon. To this suspension was added an ethereal solution of $Et_3NGaH_3$ in such amount to obtain a theoretical Ga loading of 5 % w/w of Ga with respect to the support. The ether was removed under vacuum at ca. -30 °C. After complete removal of diethyl ether, the flask was quickly (ca. 10 °C /min) heated up to 250 °C and held at this temperature until no gaseous products were developed. Once the gallane decomposition terminated, the resulting grey solid was held at this temperature under vacuum (1 mbar) overnight and then cooled and stored under argon to thereby obtain the tile compound.

**Preparation of Pd/Ga decorated material**

2 g of Ga decorated porous glass were suspended in 10 mL of distilled water under vigorous stirring. To this suspension was added a stock solution of $NH_4PdCl_4]$ (with a concentration of 2 mg $mL^{-1}$) in distilled water, in such amount to get the desired theoretical Ga/Pd ratio, once accounted for the loss of Ga due to the redox reaction between the latter and the $[PdCl_4]^{2-}$ anion. The resulting suspension was held under stirring until the reaction completed (few seconds: the orange solution turns colorless), then filtered and thoroughly washed in turn with distilled water (250 mL) and acetone (150 mL). The resulting solid was first roughly air dried and then under vacuum (1 mbar) overnight.

### 1.2 Preparation of Pt/Ga, Rh/Ga and Cr/Ga decorated materials

Triethylammonium chlorometallates of the general formula $[Et_3NH]_n[MCl_{m+n}]$ were chosen as metal precursors. These compounds were prepared according the scheme depicted below, by mixing the relevant metal chloride (Sigma-Aldrich) with a stoichiometric amounts of $[Et_3NH]Cl$ (Sigma-Aldrich) and refluxing the mixture in acetonitrile or acetonitrile/MeOH mixtures until complete dissolution:

$$n\,[Et_3NH]Cl + MCl_m \xrightarrow[\text{or } CH_3CN/MeOH]{CH_3CN} [Et_3NH]_n[MCl_{m+n}]$$

n=m=2; **M** = Pt, Ni, Cu, Co
n=1; m=3; **M** = Au, Fe, Cr, Ir, Rh, Ru

The solutions were prepared in order to obtain 0.1 g of active metal. To these metal precursor solutions, porous glass (10.0 g) was added and the solvent removed by mean of a rotavapor. The resulting solids were dried overnight at 80 °C under vacuum (0.5 mbar).

After this treatment, the obtained solids were suspended in dry ethyl ether (20 mL). The M/Ga catalyst were prepared by adding to the suspensions a 2 M solution of $Et_3NGaH_3$ in diethyl ether, in such amount to realize a final

M/Ga ratio of 10 mol/mol, once accounted for the consumed $Et_3NGaH_3$ due to the following reduction reaction:

$$[Et_3NH]_n[MCl_{n+m}] + m/3\ Et_3NGaH_3 \xrightarrow[Et_2O]{} $$

$$\xrightarrow[Et_2O]{} M^0 + (n \times m)/3\ [Et_3NH][GaCl_4] + m/2\ H_{2(g)} + m/3\ Et_3N$$

An immediate reaction took place, due to the reduction (darkening of solids) of the metals. The systems were left to react for 30 min. Afterwards the ether solution was slowly evaporated in vacuum at -30 °C in order to minimize the loss of the excess of volatile $Et_3NGaH_3$. When the solids were dry, they were heated up to 180 °C and kept at this temperature until no gas evolution was observed any longer. Then the temperature was raised up to 350 °C and the material was held at this temperature in a light Ar stream for 4 h. Then the Ar was switched off and the system held for further 2 h under vacuum (0.5 mbar). Then, the heating was turned off and the material was allowed to cool to room temperature to thereby obtain the M (Pt, Rh, Cr) / Ga decorated materials.

## 2. Catalytic reactions testing

### 2.1 setup

The catalysts are used as prepared according to the above procedure and are not reduced prior to catalytic testing. The catalytic tests were carried out in a continuous flow experimental plant at atmospheric pressure, which is depicted in figure 10. Depending on the metal loading a defined catalyst mass of 0.6 - 2.4 g is placed into the fixed-bed reactor. After a heating period of 2.5 h up to a temperature at the catalyst bed of 445 °C the reaction is started by supplying 9.3 $mL_N$ $min^{-1}$ pure n-butane (Linde, purity 2.5) as feed gas, or 9.3 $mL_N$ $min^{-1}$ n-butane diluted by 93 $mL_N$ $min^{-1}$ helium (Linde, purity 4.6) respectively, resulting in a weight hourly space velocity (WHSV) of 60 - 255 $g_{n\text{-}butane}$ $g_{Pd}^{-1}$ $h^{-1}$ and a residence time $\tau$ of 0.3 - 7.3 s.

The gases are dosed by mass flow controllers (MFC, Bronckhorst). All reactor tubes and pipes are heated by the use of heating tapes to a temperature of 180 °C and are isolated with fiberglass tape. The Alloy 600 fixed-bed reactor is heated by a heating jacket which is divided into three parts. All other parts which are in contact with the reactants are made of stainless steel 1.4571.

### 2.2 Analytic techniques

**GC**

The product stream is analyzed by an online Bruker 450 GC equipped with a ShinCarbon micropacked CP Wax 52 CB column (25 m x 0.53 mm x 0.7 mm), a HP-AL/S column (50 m x 0.535 mm x 0.015 mm) and a flame ionization detector (FID). Required mole fractions x are calculated from peak areas and calibration factors determined for every substance. The conversion of n-butane $X_{n\text{-}butane}$, turn over frequency (TOF) with regard to n-butane and selectivity to butenes $S_{butenes}$ (as the sum of 1-butene, cis-2-butene, trans-2-butene, isobutene) are calculated as follows:

$$X_{n-butane} = \frac{x_{n-butane,0} - x_{n-butane}}{x_{n-butane,0}} \cdot 100\ \% \qquad (1)$$

$$TOF = \frac{\dot{n}_{n-butane,0} \cdot X_{n-butane}}{n_{active\ Metal}} \qquad (2)$$

$$S_{butenes} = \frac{x_{butenes} - x_{butenes,0}}{x_{n-butane,0} - x_{n-butane}} \cdot 100\ \% \qquad (3)$$

The corrected conversion of n-butane $X^*_{n\text{-}butane}$ is defined as the measured conversion of the catalytic test reduced by the blank activity of the reactor $X_{n\text{-}butane}$, 0. The corrected turn over frequency (TOF*) is calculated analogous to the TOF using the corrected conversion X* instead of X.

**ICP-AES**

The content of active metal and gallium of the catalyst samples was measured by inductively coupled plasma - atom emission spectroscopy (ICP-AES) using a Ciros CCD (Spectro Analytical Instruments GmbH). The solid samples were digested in HCl:$HNO_3$:HF ratio 3:1:1 in a microwave oven at 180 °C for 40 min and the instrument was calibrated with standard solutions of the relevant substances prior to the measurements.

**2.3 Catalytic experiments**

Table 1 shows the composition of a Pd/Ga catalyst for catalytic experiments, obtained according to the procedure described above.

Table 1

| | **$n_{Ga}$/$n_{Pd}$** | mol% Ga | mol% Pd | wt%Ga | wt% Pd |
|---|---|---|---|---|---|
| diluted | **10** | 90 | 10 | 2.4 | 0.4 |
| diluted | **20** | 95 | 5 | 4.9 | 0.4 |
| non.diluted | **10.8** | 92 | 8 | 3.4 | 0.5 |
| non.diluted | **22.8** | 96 | 4 | 3.5 | 0.2 |

Table 2 shows the composition of Rh/Ga, Pt/Ga and Cr/Ga catalysts for catalytic experiments with undiluted butane feed, obtained according to the procedure described above.

Table 2

| Metal | **$n_{Ga}$/$n_{Metal}$** | mol% Ga | mol% Metal | wt%Ga | wt% Metal |
|---|---|---|---|---|---|
| Rh | 9.2 | 90 | 10 | 4.9 | 0.8 |
| Pt | 8.4 | 89 | 11 | 2.8 | 0.9 |
| Cr | 4.6 | 82 | 18 | 5.6 | 0.9 |

**Diluted experiments with the Pd/Ga catalyst:**

The following parameters were used for diluted experiments: $T_{jacket}$ = 500 °C, $T_{cat,m}$ = 445 °C, p = 1,1 bar, $\dot{V}$ butane = 9,3 $mL_N$ $min^{-1}$, $\dot{V}$ He = 93 mLN $min^{-1}$, $m_{cat}$ = 0,6 - 1,6 g, $M_{Pd,rct}$ = 0, 006 g, WHSV = 255 $g_{butane}$ $g_{pd}^{-1}$ $h^{-1}$.

The results being shown in figs. 1-3. Fig. 1 shows conversion of n-butane (%) over time (h). Fig. 2 shows the corresponding TOF* ($h^{-1}$) (TOF*: TOF corrected by blank value of reactor). Fig. 3. shows the corresponding selectivity to butenes (%).

In each of figs. 1-3 the symbols have the following meaning: a "-" represents the empty reactor, an "x" the support, an empty diamond (or triangle) Pd-coated support, an empty square the Ga-coated support. These data are included for comparison. The experiments with the inventive catalyst are marked with solid symbols: Ga/Pd=10 as solid triangle and Ga/Pd=20 solid circle.

This experiment clearly shows the superior properties of the inventive catalyst. Specifically, the combination of components A, B, and C results, in the catalytic experiments described, a significant and commercially relevant increase of catalytic activity (TOF), selectivity and life expectancy.

**Non-diluted experiments with Metal (Pd, Rh, Pt, Cr) /Ga catalyst::**

The following parameters were used for non-diluted experiments: $T_{jacket}$ = 500 °C, $T_{cat,m}$ = 445 °C, p = 1,1 bar, $\dot{V}$ butane = 9,3 mLN $min^{-1}$, $m_{cat}$ = 1 - 1,4 g, $m_{metal,rct}$ = 0, 006 -0,049 g, WHSV = 60 - 255 $g_{butane}$ $g_{Pd}^{-1}$ $h^{-1}$.

The results being shown in figs. 4 and 5 for the Pd/Ga decorated material. Figs 8 and 9 show results for Rh/Ga, Pt/Ga and Cr/Ga decorated materials.

Fig. 4 shows the TOF* ($h^{-1}$) (TOF*: TOF corrected by blank value of reactor). Fig. 5 shows the corresponding selectivity to butenes (%). In each of figs. 4 and 5 an empty triangle indicates the Pd-coated support. The experiments with the inventive catalyst are marked with solid symbols: Ga/Pd=10.8 as solid triangle and Ga/Pd=22.8 solid circle.

Fig. 8 shows the TOF* ($h^{-1}$) (TOF*: TOF corrected by blank value of reactor). Fig. 9 shows the corresponding

selectivity to butenes (%). In both figs, a solid circle represents Rh/Ga, a solid triangle represents Pt/Ga and a solid square represents Cr/Ga.

As for the diluted experiment, this non-diluted experiment clearly shows the superior properties of the inventive catalyst. Specifically, the combination of components A, B, and C results, in the catalytic experiments described, a significant and commercially relevant increase of catalytic activity (TOF), selectivity and lifetime. It is particularly to be noted that the inventive catalyst system also performs excellent under the above-described, non-diluted conditions. The prior art, e.g. as cited above, typically reports on diluted experiments. Such reports typically provide better results in a lab-scale but are of less relevance when considering commercial applications. Further, as evidenced by figs. 8 and 9, a broad variety of metals retain or improve its catalytic activity, selectivity and stability and may therefore be used as component C in the inventive catalytically active materials.

### Comparison to commercial catalysts (non-diluted)

The following parameters were used for comparative experiments: $T_{jacket}$ = 500 °C, $T_{cat,m}$ = 445 °C, p = 1,1 bar, $\dot{V}$ butane = 9,3 $mL_N$ $min^{-1}$, $m_{Kat}$ = 1 - 1,4 g, $m_{met,rct}$ = 0,006 -0, 049 g, WHSV = 60 - 255 $g_{butane}$ $g_{Pd}^{-1}$ $h^{-1}$.

Fig 6 compares the TOF* (h-1) vs. time (h) of the inventive catalyst with commercially available catalysts. Empty circle and empty triangle represent commercial CrAlOx- and Pt/AlOx-dehydriation catalysts respectively, while solid squares represent the inventive catalyst Ga/Pd= 10.

The same chromium oxide catalyst to which the performance of the inventive catalysts is compared, is also published as it was used for a cascade reaction combining dehydrogenation and hydroformylation by Walter et al. [S. Walter et al., American Institute of Chemical Engineers Journal, 2015, 61, 893-897].

The superior properties of the inventive catalytically active material are apparent.

### Long-term stability (non-diluted)

The following parameters were used for the long term experiment: $T_{jacket}$ = 500 °C, $T_{cat,m}$ = 445 °C, p = 1,1 bar, $\dot{V}$ butane = 9,3 $mL_N$ $min^{-1}$, $m_{cat}$ = 1,2 g, $m_{Pd,rct}$ = 0,006 g, WHSV = 255 $g_{butane}$ $g_{Pd}^{-1}$ $h^{-1}$

The results being shown in figs. 7 where TOF* (h-1) vs. time (h) of the inventive catalyst Ga/Pd= 10 is shown. This experiment clearly shows long-term stability of the inventive catalyst.

## Claims

1. A solid composition of matter, comprising a support and a coating, whereby
said support comprises a non-metallic and porous component (A), and
said coating comprises a first metallic component (B) and a second metallic component (C),
**characterized in that**

    • said component (C) is catalytically active and
    • said component (C) is present in component (B) in dissolved and / or in suspended form,
    • said coating has a melting point below 200°C.

2. The composition of claim 1 where
said component (A) has a specific surface of 2-10000 $m^2$/g , preferably 50-1000$m^2$/g; and / or is thermally stable up to 400°C, preferably up to 800°C; and/or said component (B) has a melting point below 200°C; and / or said component (C)has a solubility of at least 0.01 mass% in component (B).

3. The composition according to any of the preceding claims, where
said component (A) is selected from the group consisting of metal oxides, silicates, carbonaceous materials, preferably aluminium oxide and silica; and / or
said component (B) is a metal selected from the group consisting of Ga, In, and Hg or an alloy selected from the group consisting of Ga/In, Wood's metal or Rose's metal, preferably Ga and Ga containing alloys; and / or
said component (C) selected from the group of metals as defined by IUPAC group 3 to 12, particularly Pd, Pt, Rh, Cr; and from the group of alloys comprising a metal as defined by IUPAC group 3 to 12, particularly Pt/Co, Pt/Sn, Pt/Re, Pd/Zn.

4. The composition according to any of the preceding claims, wherein said support is

- in the form of a powder;
- in the form of granulated material;
- in the form of spheres; or
- in the form of a shaped article.

**5.** The composition according to any of the preceding claims, wherein said coating

- fully covers said support, preferably with a film thickness of at least 0.4 nm; or
- partly covers said support, preferably to more than 1 Area-%, such as 10 Area-%.

**6.** The composition according to any of the preceding claims, wherein

- the amount of [(C)+(B)]:(A) is from about 0.01 to about 300 weight percent based on the total weight of the support; and / or
- the amount of (B) to (C) is from about 1:1 to 1000:1.

**7.** A method for manufacturing a composition according to any of the preceding claims, comprising the steps of

(i) providing a component (A); a solution comprising a precursor of (B) and a solution comprising a precursor of (C)
(ii) contacting said component (A) with said solutions comprising precursors of (B) and (C) either simultaneously or subsequently, removing solvent and
(iii) optionally further activation steps;

to thereby obtain said solid composition of matter.

**8.** The method of claim 7 wherein

- said precursor of (B) is selected from the group of alkylammonium gallates; and/or
- said precursor of (C) is selected from the group of alkylammonium or ammonium halometallates.

**9.** A solid composition, particularly a catalyst composition, obtained by a method according to any of claims 7 - 8.

**10.** Use of a composition according to any of claims 1-6 as a catalyst, particularly as a catalyst in a gas-phase reaction.

**11.** The use according to claim 10, where said catalysed reaction is selected from the group of endothermic reactions.

**12.** A catalytic process, comprising the step of contacting an organic starting material with a composition according to any of claims 1-6, where

- reaction temperatures are above 200°C; and/or
- reaction cycles are at least 1 hour; and/or
- starting materials and reaction products of the catalytic process are gaseous under reaction conditions.

**13.** The catalytic process according to claim 12, said process being selected from the group of

- catalytic isomerisation of hydrocarbons or of aromatics,
- catalytic dehydrogenation of hydrocarbons,
- catalytic reforming.

**14.** The catalytic process according to claims 12 and 13, the starting material being selected from the group consisting of aliphatic or aromatic hydrocarbons, including linear, branched and cyclic C2+ alkanes or C6+ aromatics, in particular ethane, propane, butane, isobutane, pentane, isopentanes, hexane, isohexanes, cyclohexane, heptane, isoheptanes, methylcyclohexane, ethyl benzene, perhydro benzyltoluene, perhydro dibenzyltoluene and mixtures thereof.

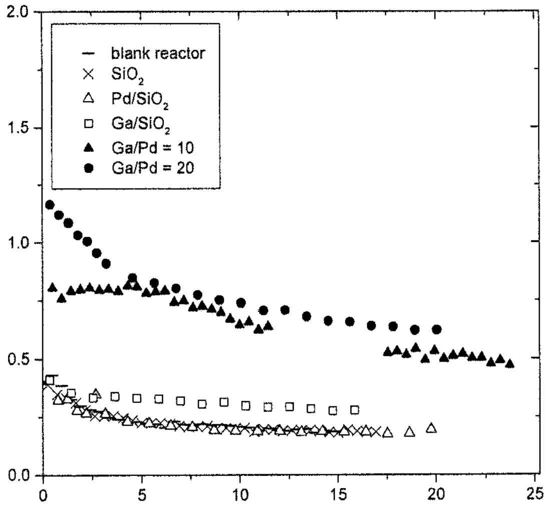


fig. 1

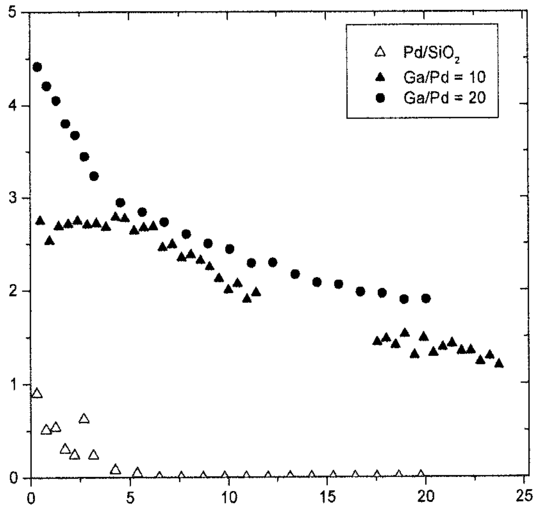


fig. 2

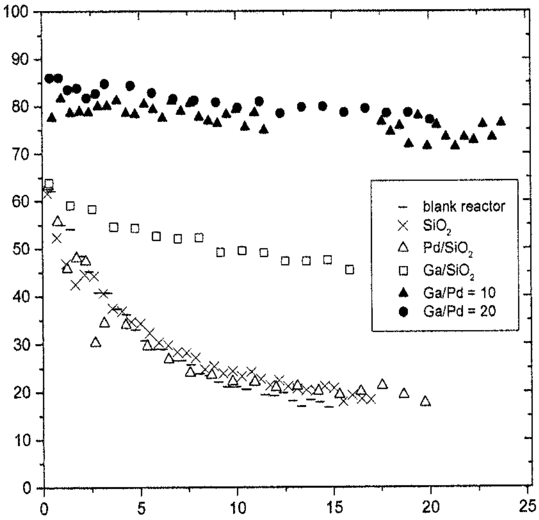


fig.3

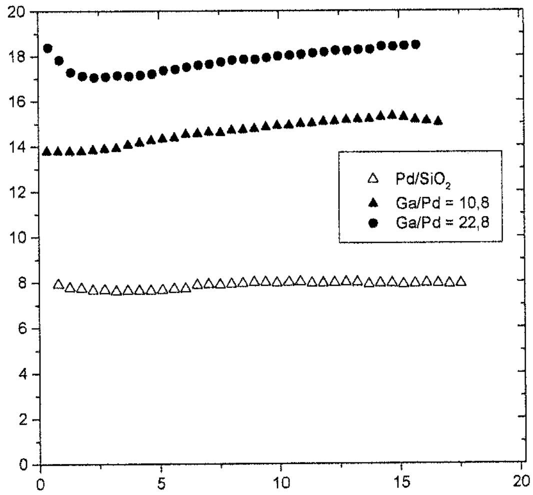


fig. 4

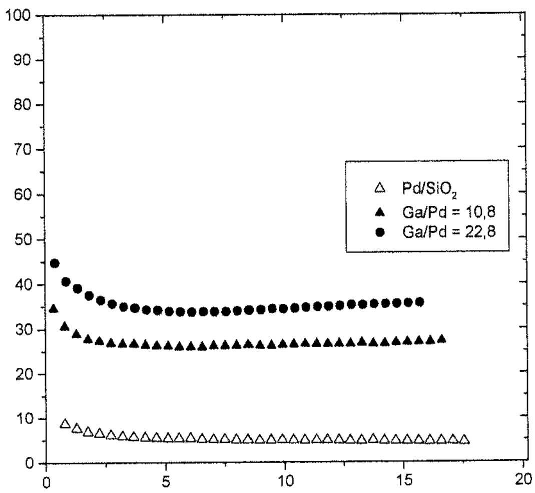


fig. 5

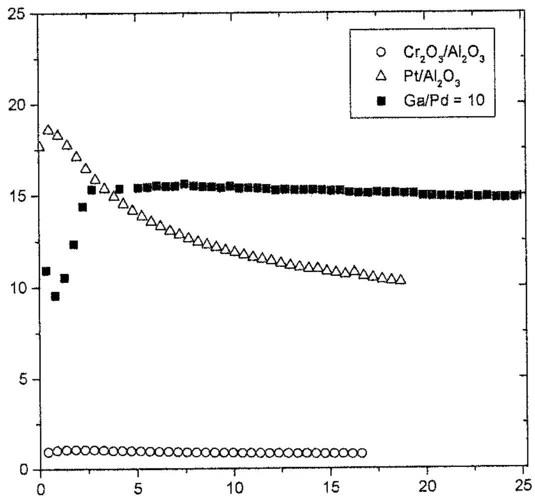


fig. 6

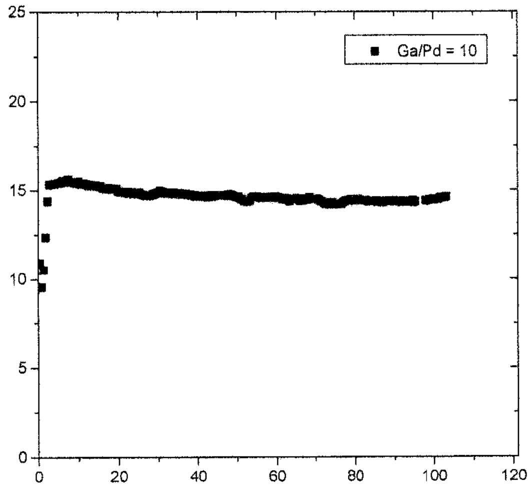


fig. 7

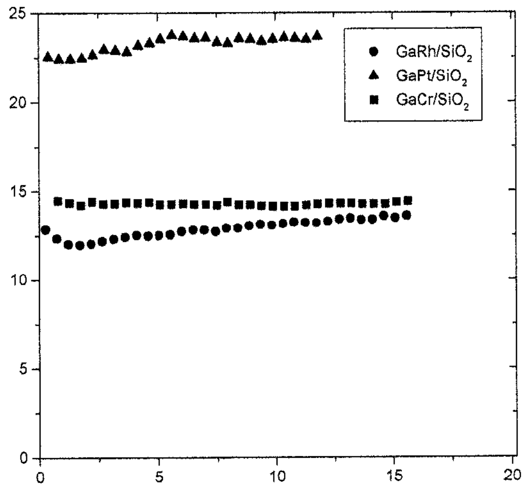


fig. 8

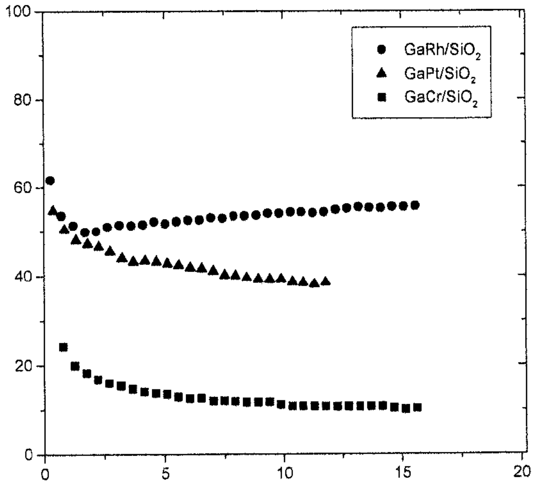


fig. 9

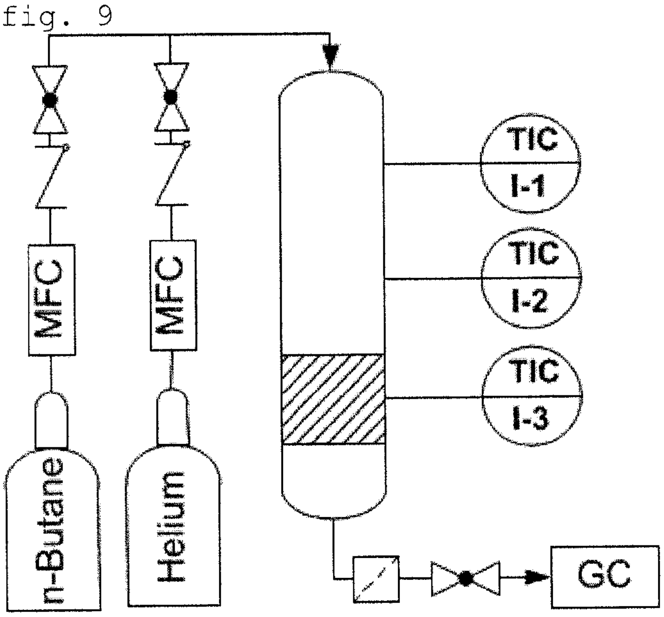


fig.10

Europäisches Patentamt
European Patent Office
Office européen des brevets

# EUROPEAN SEARCH REPORT

Application Number
EP 16 18 5958

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 822 699 A (WAN CHUNG-ZONG [US]) 18 April 1989 (1989-04-18)<br>* abstract *<br>* column 2, lines 18-39 *<br>* examples 2,6 *<br>----- | 1-10,12 | INV.<br>B01J23/26<br>B01J23/62<br>B01J35/00<br>B01J37/02<br>B01J38/12<br>B01J38/10<br>B01J23/96<br>C07C5/00 |
| X | US 2010/236985 A1 (LUO LIN [US] ET AL) 23 September 2010 (2010-09-23)<br>* abstract *<br>* paragraphs [0103] - [0106] *<br>----- | 1-14 | |
| X | JESPER J. H. B. SATTLER ET AL: "Catalytic Dehydrogenation of Light Alkanes on Metals and Metal Oxides",<br>CHEMICAL REVIEWS,<br>vol. 114, no. 20,<br>22 October 2014 (2014-10-22), pages 10613-10653, XP055237672,<br>US<br>ISSN: 0009-2665, DOI: 10.1021/cr5002436<br>* abstract *<br>* page 10636, left-hand column, line 5 - right-hand column, line 7 *<br>* table 7 *<br>----- | 1-14 | TECHNICAL FIELDS SEARCHED (IPC)<br>B01J<br>C07C |
| X | US 4 224 192 A (FOSTER ALAN I ET AL) 23 September 1980 (1980-09-23)<br>* abstract *<br>* column 2, lines 22-35 *<br>* column 3, lines 9-11 *<br>* examples 2,5 *<br>----- | 1-7,9-14 | |
| X | US 2007/123418 A1 (HAN HYUN-SIK [KR] ET AL) 31 May 2007 (2007-05-31)<br>* abstract *<br>* figure 1 *<br>-----<br>-/-- | 1-10 | |

1

The present search report has been drawn up for all claims

| Place of search | Date of completion of the search | Examiner |
|---|---|---|
| Munich | 1 March 2017 | Fischbach, Malaika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 16 18 5958

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/303713 A1 (ZHANG QINGLIN [US] ET AL) 2 December 2010 (2010-12-02)<br>* abstract *<br>* paragraphs [0013] - [0018] *<br>* example 3 *<br>----- | 1-10,12 | |
| X | US 2010/216630 A1 (GAJDA GREGORY J [US] ET AL) 26 August 2010 (2010-08-26)<br>* abstract *<br>* examples 1,2 *<br>----- | 1-14 | |
| X | DE 10 2009 045804 A1 (INST MIKROTECHNIK MAINZ GMBH [DE])<br>21 April 2011 (2011-04-21)<br>* abstract *<br>* examples 1,2 *<br>----- | 1-10,12,13 | |
| X | EP 1 533 029 A1 (ROHM & HAAS [US])<br>25 May 2005 (2005-05-25)<br>* abstract *<br>* example 2 *<br>* tables 1,3 *<br>----- | 1-14 | TECHNICAL FIELDS SEARCHED (IPC) |
| X | US 4 469 812 A (SORRENTINO CECELIA M [US] ET AL) 4 September 1984 (1984-09-04)<br>* abstract *<br>* examples 1,2 *<br>----- | 1-14 | |
| X | US 4 880 711 A (LUCZAK FRANCIS J [US] ET AL) 14 November 1989 (1989-11-14)<br>* abstract *<br>* examples 1,2 *<br>----- | 1-7 | |

The present search report has been drawn up for all claims

1

| Place of search | Date of completion of the search | Examiner |
|---|---|---|
| Munich | 1 March 2017 | Fischbach, Malaika |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 16 18 5958

This annex lists the patent family members relating to the patent documents cited in the above-mentioned European search report. The members are as contained in the European Patent Office EDP file on
The European Patent Office is in no way liable for these particulars which are merely given for the purpose of information.

01-03-2017

| Patent document cited in search report | Publication date | Patent family member(s) | Publication date |
|---|---|---|---|
| US 4822699 A | 18-04-1989 | NONE | |
| US 2010236985 A1 | 23-09-2010 | AR 075889 A1 | 04-05-2011 |
| | | BR PI1006446 A2 | 31-01-2017 |
| | | CN 102355947 A | 15-02-2012 |
| | | CN 105859503 A | 17-08-2016 |
| | | EP 2408558 A1 | 25-01-2012 |
| | | US 2010236985 A1 | 23-09-2010 |
| | | WO 2010107591 A1 | 23-09-2010 |
| US 4224192 A | 23-09-1980 | NONE | |
| US 2007123418 A1 | 31-05-2007 | NONE | |
| US 2010303713 A1 | 02-12-2010 | AU 2010254196 A1 | 22-12-2011 |
| | | CA 2763124 A1 | 02-12-2010 |
| | | CN 102802785 A | 28-11-2012 |
| | | EP 2435182 A2 | 04-04-2012 |
| | | JP 5678041 B2 | 25-02-2015 |
| | | JP 2012528007 A | 12-11-2012 |
| | | KR 20120024841 A | 14-03-2012 |
| | | RU 2011152487 A | 10-07-2013 |
| | | US 2010303713 A1 | 02-12-2010 |
| | | US 2016023193 A1 | 28-01-2016 |
| | | WO 2010138483 A2 | 02-12-2010 |
| | | ZA 201109481 B | 29-10-2014 |
| US 2010216630 A1 | 26-08-2010 | BR PI1007999 A2 | 08-03-2016 |
| | | CN 102413924 A | 11-04-2012 |
| | | RU 2011138946 A | 27-03-2013 |
| | | SG 173702 A1 | 29-09-2011 |
| | | US 2010216630 A1 | 26-08-2010 |
| | | WO 2010096336 A2 | 26-08-2010 |
| DE 102009045804 A1 | 21-04-2011 | DE 102009045804 A1 | 21-04-2011 |
| | | EP 2490804 A1 | 29-08-2012 |
| | | US 2012207667 A1 | 16-08-2012 |
| | | WO 2011047968 A1 | 28-04-2011 |
| EP 1533029 A1 | 25-05-2005 | BR PI0404901 A | 19-07-2005 |
| | | CN 1636632 A | 13-07-2005 |
| | | EP 1533029 A1 | 25-05-2005 |
| | | JP 2005144432 A | 09-06-2005 |
| | | JP 2008100226 A | 01-05-2008 |
| | | KR 20050048528 A | 24-05-2005 |
| | | TW I279253 B | 21-04-2007 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 16 18 5958

This annex lists the patent family members relating to the patent documents cited in the above-mentioned European search report. The members are as contained in the European Patent Office EDP file on The European Patent Office is in no way liable for these particulars which are merely given for the purpose of information.

01-03-2017

| Patent document cited in search report | Publication date | Patent family member(s) | Publication date |
|---|---|---|---|
| | | US 2005131255 A1 | 16-06-2005 |
| US 4469812 A | 04-09-1984 | NONE | |
| US 4880711 A | 14-11-1989 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **J. SATTLER et al.** *Chemical Reviews,* 2014, vol. 114, 10613-10653
- **S. VELDURTHI et al.** *Catalysis Today,* 2012, vol. 185, 88-93
- **S. BOCANEGRA et al.** *Catalysis Today,* 2009, vol. 143, 334-340
- **J. SATTLER et al.** *Physical Chemistry Chemical Physics,* 2013, 12095-12103
- **SHRIVER et al.** *Inorg. Synth.,* 1977, vol. 17, 42-45
- **S. WALTER et al.** *American Institute of Chemical Engineers Journal,* 2015, vol. 61, 893-897